# EUROPÄISCHE PATENTANMELDUNG

(11) **EP 4 198 003 A1**
(43) Veröffentlichungstag der Anmeldung: **21.06.2023**
(21) Anmeldenummer: 22212189.9
(22) Anmeldetag: 08.12.2022
(51) Int. Cl.: C07C 45/00, C07F 15/00, B01J 31/00, C07B 41/06

(54) **PT-THIXANTPHOS-IOD-KOMPLEX UND PT-THIXANTPHOS-BROM-KOMPLEX**

(30) Priorität: 17.12.2021 EP 21215344
(71) Anmelder: Evonik Operations GmbH, 45128 Essen (DE)
(72) Erfinder: Schneider, Carolin, 40789 Monheim am Rhein (DE); Jackstell, Ralf, 18106 Rostock (DE); Beller, Matthias, 18211 Ostseebad Nienhagen (DE); Franke, Robert, 45772 Marl (DE)
(74) Vertreter: Evonik Patent Association

(57) **Zusammenfassung**

Pt-Thixantphos-Iod-Komplex und Pt-Thixantphos-Brom-Komplex, sowie deren Verwendung zur Katalyse einer Hydroformylierungsreaktion.

## Beschreibung

Die vorliegende Erfindung betrifft einen Pt-Thixantphos-Iod-Komplex und Pt-Thixantphos-Brom-Komplex, sowie deren Verwendung zur Katalyse einer Hydroformylierungsreaktion.

In P. Meessen et al., Journal of Organometallic Chemistry, 551, (1998), 165-170 wird der Einsatz von ThixantphosPtCl₂ zur Hydroformylierung von Methyl-3-pentenoat beschrieben.

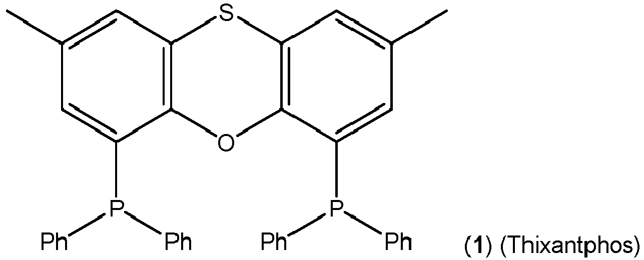

Der vorliegende Erfindung lag die Aufgabe zugrunde, einen neuen Komplex bereitzustellen. Der Komplex soll hierbei bei der Katalyse von Hydroformylierungsreaktionen eine gute Ausbeute liefern.

Diese Aufgabe wird gelöst durch einen Komplex gemäß Anspruch 1.

Komplex umfassen:
a) Pt;
b) einen Liganden entsprechend der Formel (**I**): 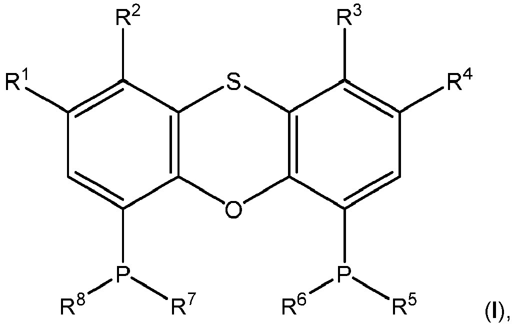 wobei R¹, R², R³, R⁴, R⁵, R⁶, R⁷, R⁸ ausgewählt sind aus: -H, -(C₁-C₁₂)-Alkyl, -(C₆-C₂₀)-Aryl;
c) einen Iod-Liganden oder Brom-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, iso-Butyl, sec-Butyl, *tert*-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform stehen R⁵, R⁶, R⁷, R⁸ für -(C₆-C₂₀)-Aryl.

In einer Ausführungsform stehen R⁵, R⁶, R⁷, R⁸ für -Ph.

In einer Ausführungsform stehen R¹ und R⁴ für -(C₁-C₁₂)-Alkyl.

In einer Ausführungsform stehen R¹ und R⁴ für -CH₃.

In einer Ausführungsform stehen R² und R³ für -H.

In einer Ausführungsform stehen weist die Verbindung gemäß der Formel (**I**) die Struktur **(1)** auf:

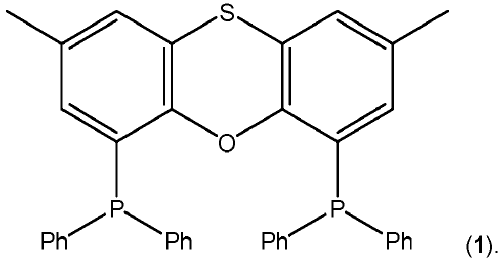

In einer Ausführungsform weist der Komplex genau einen Liganden entsprechend der Formel (**I**) auf.

In einer Ausführungsform weist der Komplex mindestens zwei Iod-Liganden auf.

In einer Ausführungsform weist der Komplex genau zwei Iod-Liganden auf.

In einer Ausführungsform weist der Komplex mindestens zwei Brom-Liganden auf.

In einer Ausführungsform weist der Komplex genau zwei Brom-Liganden auf.

Neben dem Komplex an sich, wird auch dessen Verwendung zur Katalyse einer Hydroformylierungsreaktion beansprucht.

Verwendung eines zuvor beschriebenen Komplexes zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 18 h bei 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Hydroformylierung von 1-Octen

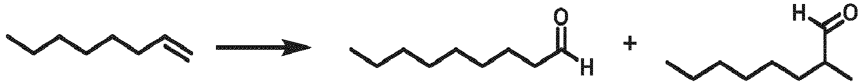

Reaktionsbedingungen:
1.0 mmol 1-Octen, 0.5 mol% PtI₂, 2,0 Äquivalente Ligand, Lösungsmittel: Toluol, p(CO/H₂): 40 bar, T: 80 °C, t: 18 h.

| Ligand | Ausbeute (%) |
|---|---|
| 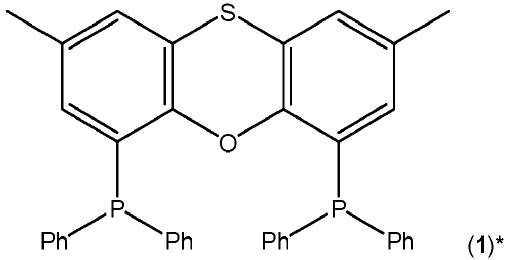 | PtI₂*: 99 |

| | |
|---|---|
| * erfindungsgemäßes Verfahren | |

### Variation des Halogens

Reaktionsbedingungen:
10 mmol 1-Octen, 0.1 mol% PtX₂, 2,2 Äquivalente Ligand (1), Lösungsmittel: Toluol, p(CO/H₂): 40 bar, T: 80 °C, t: 20 h.

Ausbeuten:
PtI₂: 95%
PtBr₂: 48%
PtCl₂: < 1%

Wie die Versuchsergebnisse zeigen, wird die Aufgabe durch den erfindungsgemäßen Komplex gelöst.

## Patentansprüche

1. Komplex umfassen:
a) Pt;
b) einen Liganden entsprechend der Formel (**I**): 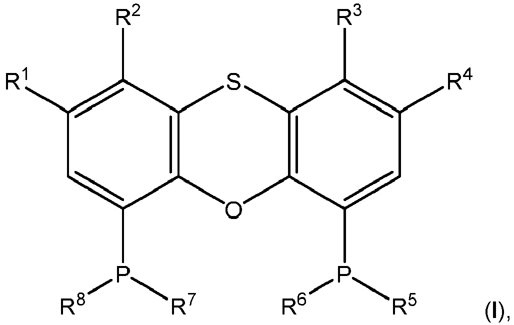 wobei R¹, R², R³, R⁴, R⁵, R⁶, R⁷, R⁸ ausgewählt sind aus: -H, -(C₁-C₁₂)-Alkyl, -(C₆-C₂₀)-Aryl;
c) einen Iod-Liganden oder Brom-Liganden.

2. Komplex nach Anspruch 1,
wobei R⁵, R⁶, R⁷, R⁸ für -(C₆-C₂₀)-Aryl stehen.

3. Komplex nach einem der Ansprüche 1 oder 2,
wobei R⁵, R⁶, R⁷, R⁸ für -Ph stehen.

4. Komplex nach einem der Ansprüche 1 bis 3,
wobei R¹ und R⁴für -(C₁-C₁₂)-Alkyl stehen.

5. Komplex nach einem der Ansprüche 1 bis 4,
wobei R² und R³ für -H stehen.

6. Komplex nach einem der Ansprüche 1 bis 5,
wobei die Verbindung gemäß der Formel (**I**) die Struktur **(1)** aufweist:

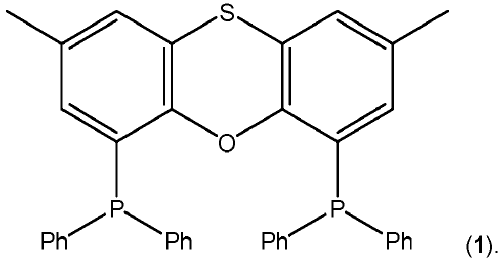

7. Komplex nach einem der Ansprüche 1 bis 6,
wobei der Komplex genau einen Liganden entsprechend der Formel (**I**) aufweist.

8. Komplex nach einem der Ansprüche 1 bis 7,
wobei der Komplex mindestens zwei Iod-Liganden aufweist.

9. Komplex nach Anspruch 8,
wobei der Komplex genau zwei Iod-Liganden aufweist.

10. Komplex nach einem der Ansprüche 1 bis 7,
wobei der Komplex mindestens zwei Brom-Liganden aufweist.

11. Komplex nach Anspruch 10,
wobei der Komplex genau zwei Brom-Liganden aufweist.

12. Verwendung eines Komplexes nach einem der Ansprüche 1 bis 11 zur Katalyse einer Hydroformylierungsreaktion.
